## Europäisches Patentamt

## European Patent Office

## Office européen des brevets

(19)

(11) Veröffentlichungsnummer: **0 187 328**
**A1**

(12) # EUROPÄISCHE PATENTANMELDUNG

(21) Anmeldenummer: **85116180.2**

(22) Anmeldetag: **18.12.85**

(51) Int. Cl.⁴: **A 61 H 9/00**
**//A61M1/06**

(30) Priorität: 24.12.84 CH 6138/84

(43) Veröffentlichungstag der Anmeldung:
16.07.86 Patentblatt 86/29

(84) Benannte Vertragsstaaten:
DE FR GB SE

(71) Anmelder: I S G AG
Alpenstrasse 2
CH-6300 Zug(CH)

(72) Erfinder: Larsson, Karl Olof (Olle) Axel Helmer
Lindenweg 2
CH-6300 Zug(CH)

(74) Vertreter: Blum, Rudolf Emil Ernst et al,
c/o E. Blum & Co Patentanwälte Vorderberg 11
CH-8044 Zürich(CH)

(54) Gerät zur mechanischen Reizung der Brustwarzen einer schwangeren Frau.

(57) Das Gerät hat ein glockenförmiges Traggerüst (1-3), dessen Wandung (3) grossflächige Durchbrüche (4) aufweist. Das Traggerüst (1-3) ist von einer gummielastischen Membrane (6) umgeben und bildet mit dieser zusammen eine Haube, die mit einem Saugleitungsanschluss (5) versehen ist. Wenn das Gerät über eine Brustwarze der Frau gestülpt wird und im Innern des Gerätes ein intermittierender Unterdruck aufgebaut wird, wird die gummielastische Membrane (6) durch die Durchbrüche (4) hindurch nach innen gezogen und wirkt dabei intermittierend auf die Brustwarze und deren Umgebung. Dieses sanfte, weiche Drücken und Saugen der Brustwarze ergibt die gewünschte Reizung.

*Fig. 1*

- 1 -

## Gerät zur mechanischen Reizung der Brustwarzen
## einer schwangeren Frau

Die vorliegende Erfindung betrifft ein Gerät zur mechanischen Reizung der Brustwarzen einer schwangeren Frau.

Untersuchungen haben ergeben, dass durch eine sanfte Reizung der Brustwarzen und deren direkter Umgebung die Produktion gewisser Hormone bzw. anderer Stoffe hervorgerufen bzw. gefördert wird. So wird z.B. durch die genannte Reizung die Produktion eines Stoffes angeregt, bzw. gefördert, welcher bei schwangeren Frauen die Wehentätigkeit auslöst bzw. verstärkt. Diese Wehentätigkeit bzw. verstärkte Wehentätigkeit ist von Bedeutung bei Vorgängen wie Beschleunigung der Entbindung, Einleitung der Entbindung, Abstossen der Placenta. Ferner werden vorgeburtliche Untersuchungen am Ungeborenen (Kontraktion-Stress-Tests) durchgeführt, und in diesem Zusammenhang werden Wehen provoziert. Weiter hat sich gezeigt, dass durch denselben Vorgang auch die Produktion von Muttermilch gefördert wird.

Diese Vorgänge werden zur Zeit durch pharmakologische Stoffe ausgelöst. Um diese pharmakologischen Stoffe zu umgehen und den Körper zur Produktion der notwendigen Hormone bzw. Stoffe auf natürlichem Weg zu bringen, hat sich nun die Aufgabe gestellt, ein geeignetes Gerät hierfür zur Verfügung zu stellen.

Feu/lb
10.12.1985                                        EU 1278

Zweck der vorliegenden Erfindung ist somit die Schaffung eines Gerätes, welches die Brustwarzen der schwangeren Frau auf einfache und sanfte Weise zu reizen vermag. Selbstverständlich soll das Gerät einfach im Aufbau und im Betrieb sein.

Das Gerät zeichnet sich erfindungsgemäss aus durch ein haubenförmiges, eigensteifes Traggerüst, wobei die Haubenwand mit grossflächigen Durchbrüchen versehen ist, eine auf der Aussenseite des Gerüstes angeordnete, über den Durchbrüchen liegende Membrane und durch einen Saugleitungsanschluss, über welchen der Haubeninnenraum mit einer intermittierend arbeitenden Sauganlage verbindbar ist.

Die Erfindung wird nachstehend anhand der Beschreibung eines in der Zeichnung dargestellten Ausführungsbeispiels noch etwas näher erläutert. Es zeigt:

Fig. 1 einen Schnitt durch ein glockenförmiges Gerät nach der Erfindung, und

Fig. 2 eine Draufsicht auf das Gerät nach Fig. 1.

Nachstehend wird ein Gerät beschrieben, welches zur mechanischen Reizung der Brustwarzen einer schwangeren Frau dient, um dabei den Körper der Frau zur Produktion von Hormonen oder anderen Stoffen anzuregen.

Das in der Zeichnung gezeigte Gerät besteht aus einem glockenförmig ausgebildeten Traggerüst aus Kunststoff, welches neben einem Basisring 1 aus dem Bodenteil 2 und den Verbindungsstegen 3 besteht. Zwischen den Stegen 3 sind vier grossflächige Durchbrüche 4 vorhanden, und am oberen Ende des Bodenteils 2 ist ein Anschlussstutzen 5 für eine Saugleitung (nicht dargestellt) vorhanden. Die Saugleitung führt zu einer intermittierend arbeitenden Pumpe, welche im Innenraum des Gerätes intermittierend einen Unterdruck schafft. Als Pumpe eignet sich beispielsweise eine für das Absaugen von Muttermilch geeignete Pumpe.

Auf der Aussenseite des Traggerüstes ist eine ebenfalls glockenförmige Hülle 6 aus gummielastischem Material, z.B. aus durchsichtigem Gummi angeordnet. Die Hülle 6 schliesst dabei die Durchbrüche 4 dicht ab und umgibt zudem den unteren Rand des Ringes 1 und bildet dort ein nachgiebiges Polster 7. Das Traggerüst 1-3 mit der umgebenden Hülle 6 ergibt eine auf die Brust der Frau aufsetzbare Haube, die flexible Wandteile (die Hülle 6 über den Durchbrüchen 4) aufweist. Da durch leichtes Andrücken des Gerätes an der Brust ein luftdichter Verschluss erfolgt, entsteht im Betrieb im Inneren der Haube ein Unterdruck, welcher bewirkt, dass die elastische Membrane 6 durch die Durchbrüche 4 hindurch ins Innere gezogen wird. Dabei wird durch das Membranenmaterial intermittierend die eigentliche Brustwarze und deren Vorhof mechanisch gestreichelt und damit gereizt.

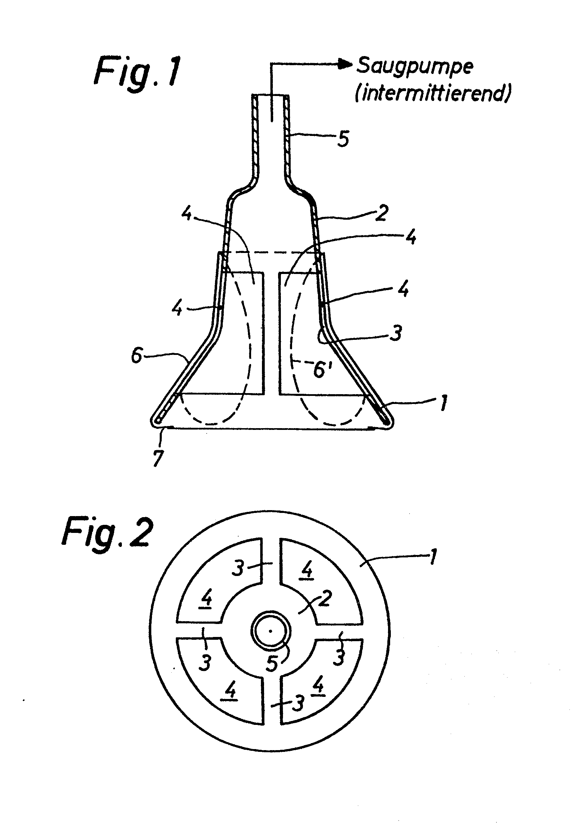

Durch die Figuren 1 und 2 ist ersichtlich, dass die vier Durchbrüche 4 den grössten Flächenteil der Mantelfläche des haubenförmigen Traggerüstes 1,2 und 3 umfassen, so dass benachbarte Durchbrüche 4 nur noch durch die Stege 3 voneinander getrennt liegen, wie aus Figur 2 ersichtlich ist. Aus Figur 1 und 2 ist weiterhin ersichtlich, dass die Durchbrüche 4 eine Rechteckkontur haben.

Patentansprüche

1. Gerät zur mechanischen Reizung der Brustwarzen einer schwangeren Frau, gekennzeichnet, durch ein haubenförmiges, eigensteifes Traggerüst (1-3), wobei die Haubenwand (3) mit grossflächigen Durchbrüchen (4) versehen ist, eine auf der Aussenseite des Gerüstes angeordnete, über den Durchbrüchen (4) liegende Membrane (6) und durch einen Saugleitungsanschluss (5), über welchen der Haubeninnenraum mit einer intermittierend arbeitenden Sauganlage verbindbar ist.

2. Gerät nach Anspruch 1, dadurch gekennzeichnet, dass das Traggerüst (1-3) als Glocke ausgebildet ist, welche aus einem ringförmigen Randteil (1) und einem über Stege (3) mit diesem verbundenen Bodenteil (2) besteht, wobei im Bodenteil (2) ein Anschlussstutzen (5) für eine Saugleitung vorhanden ist.

3. Gerät nach Anspruch 1, dadurch gekennzeichnet, dass die Membrane (6) eine über das Traggerüst (1-3) gestülpte und von aussen daran anliegende Hülle aus gummielastischem Material ist, welche einen luftdichten Verschluss der Durchgänge (4) gewährleistet.

4. Gerät nach Anspruch 3, dadurch gekennzeichnet, dass die Hülle (6) den unteren Rand des Traggerüstes (1-3) umgreift und dabei ein Schutzpolster bildet.

Gerät nach Anspruch 1, dadurch gekennzeichnet, dass die Membrane (6) auswechselbar angeordnet ist.

6. Gerät nach Anspruch 1, dadurch gekennzeichnet, dass die Durchbrüche (4) den grössten Flächenteil der Mantelfläche des haubenförmigen Traggerüstes (1-3) umfassen, so dass benachbarte Durchbrüche (4) nur noch durch Stege (3) voneinander getrennt liegen, und dass die Durchbrüche (4) eine Rechteckkontur haben.

7. Verwendung des Gerätes nach einem der Ansprüche 1 bis 6 zum Produzieren von Hormonen und anderen Stoffen, um bei der schwangeren Frau vorgeburtliche Untersuchungen vorzunehmen oder um den Geburtsvorgang zu fördern.

## Fig.1

Saugpumpe
(intermittierend)

## Fig.2

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | FR-A- 599 054 (C. PETIT et al.) <br> * Figuren 5,6; Seite 1, Zeilen 38-45; Seite 2, Zeilen 88-101 * | 1,3,5 | A 61 H 9/00 // <br> A 61 M 1/06 |
| A | GB-A- 17 778 (T. FLETCHER)(A.D. 1915) | 1 | |
| A | US-A-2 000 710 (C. MILLER) | 1 | |
| A | DE-C- 540 934 (C. PFEIFFER) | 1,2 | |
| A | BE-A- 831 799 (F. REMY) | 1 | |
| A | GB-A- 168 234 (THE RIDD CO.) | 1 | |

RECHERCHIERTE
SACHGEBIETE (Int Cl 4)

A 61 M
A 01 J

Der vorliegende Recherchenbericht wurde für alle Patentansprüche erstellt

| Recherchenort | Abschlußdatum der Recherche | Prüfer |
|---|---|---|
| DEN HAAG | 17-04-1986 | VEREECKE A. |